# EUROPÄISCHE PATENTSCHRIFT

(11) **EP 2 431 058 B1**
(45) Veröffentlichungstag und Bekanntmachung des Hinweises auf die Patenterteilung: **12.05.2021**
(21) Anmeldenummer: 11181565.0
(22) Anmeldetag: 16.09.2011
(51) Int. Cl.: A61L 2/18, A61L 2/22

(54) **Aseptik-Sterilisationseinheit für Reinraum**
Aseptic sterilisation unit for clean room
Unité de stérilisation aseptisée pour une salle blanche

(30) Priorität: 20.09.2010 DE 102010045832
(43) Veröffentlichungstag der Anmeldung: 21.03.2012
(73) Patentinhaber: Krones AG, 93073 Neutraubling (DE)
(72) Erfinder: Voth, Klaus, 93073 Neutraubling (DE)
(74) Vertreter: Bittner, Bernhard

(56) Entgegenhaltungen:
- EP-A1- 2 246 176
- EP-A1- 2 368 835
- EP-A2- 2 388 127
- DE-U1-202009 010 813

## Beschreibung

Die Erfindung betrifft eine Vorrichtung zum Behandeln von Kunststoffbehältnissen, wobei die Vorrichtung mindestens einen beweglichen Träger aufweist, welcher in einen Isolatorraum angeordnet ist, zu dem ein Sterilisationsmittel zuführbar ist. Außerdem betrifft die Erfindung ein Verfahren zur Sterilisierung eines Isolatorraumes einer Vorrichtung zum Behandeln von Kunststoffbehältnissen mit mindestens einen beweglichen Träger, welcher in einen Isolatorraum angeordnet ist, zu dem ein Sterilisationsmittel zugeführt wird.

Vorrichtungen zum Behandeln von Kunststoffbehältnissen sind aus dem Stand der Technik seit langem bekannt. Ein Beispiel für eine derartige Vorrichtung sind Blasmaschinen zum Umformen von Kunststoffvorformlingen zu Kunststoffbehältnissen. Andere derartige Vorrichtungen sind beispielsweise Ofen (z. B. Mikrowellenöfen) zum Erwärmen von Behältnissen oder Transportsterne für den Transport der Kunststoffbehältnisse. Üblicherweise weisen solche Vorrichtungen mindestens einen beweglichen Träger auf, an dem Einrichtungen zum Aufnehmen der Kunststoffbehältnisse angeordnet sind. Blasmaschinen weisen beispielsweise in der Regel eine Vielzahl von beweglichen Blasformen auf, welche jeweils einen Hohlraum ausbilden, innerhalb dessen die Kunststoffvorformlinge zu Behältnissen umformbar sind und innerhalb derer Vorformlinge aus Kunststoff (Kunststoffvorformlinge) zu Kunststoffbehältnissen expandiert werden. Derartige Blasmaschinen sind üblicherweise rotierende, etwa kreisförmige Anlagen. Dabei nehmen sie Kunststoffvorformlinge an einer bestimmten Position auf und formen während der Rotation und des Überschreitens eines bestimmten Sektors aus diesen die Kunststoffbehältnisse, die wiederum an einer bestimmten Position abgeführt werden.

DE 20 2009 010813 U1 offenbart eine Vorrichtung zum Sterilisieren von Gegenständen insbesondere Verpackungen, Behältnissen und/oder Maschinenteilen, die mindestens einen Bearbeitungsraum zum Anordnen von mindestens einem der Gegenstände aufweist, der Bearbeitungsraum zumindest durch einen unbewegten Wandungsbereich und einen bewegten Wandungsbereich begrenzt ist, mindestens eine Zuführeinrichtung zum Zuführen eines fließfähigen Desinfektionsmittels, ein bevorzugt gasförmiges Desinfektionsmittel und besonders bevorzugt Wasserstoffperoxyd, in dem Bearbeitungsraum aufweist und mindestens eine Transporteinrichtung zum Transportieren des Gegenstandes umfasst, wobei der Gegenstand zumindest zeitweise innerhalb des Bearbeitungsraums transportierbar ist.

EP 2 368 835 offenbart eine Vorrichtung (1) zum Behandeln von Behältnissen, mit einem Behandlungsraum (2), in welchem die Behältnisse (10) behandelt werden, mit einer Transporteinrichtung (4), welche die Behältnisse (10) innerhalb des Behandlungsraums (2) transportiert, wobei der Behandlungsraum durch Düsen mit Desinfektionsmittel beaufschlagt wird.

Die EP 2 388 127 offenbart eine Vorrichtung zum Umformen von Kunststoffvorformlingen mit einem Sterilraum. Dabei ist ein Bereich einer Transporteinrichtung zum Transportieren von Blasstationen außerhalb dieses Sterilraums angeordnet.

Die EP 2 246176 offenbart ebenfalls eine Vorrichtung zum Blasformen von Behältnissen mit einem Reinraum. Dabei sind Anntriebskomponenten außerhalb dieses Sterilraumes angeordnet.

An derartige Blasmaschinen aber auch andere Vorrichtungen zum Behandeln von Kunststoffbehältnissen werden stetig höhere Ansprüche in Bezug auf den Durchsatz und Qualität der produzierten bzw. behandelten Gebinde gestellt. Insbesondere die Hygiene und damit die Keimfreiheit der Gebinde ist in dem Bereich der Abfüllung von Lebensmitteln gefragt. Durch die Sterilisation der verhältnismäßig kleinen Kunststoffvorformlinge und die anschließende sterile (aseptische) Behandlung dieser Kunststoffvorformlinge über den Blasprozess zu dem gewünschten Gebinde bis zum Abfüllvorgang ist es möglich, den Gesamtprozess nicht nur zu beschleunigen, sondern auch die Menge an eingesetztem Sterilisationsmittel zu reduzieren, da lediglich die Oberfläche der gegenüber den fertigen Gebinden deutlich kleineren Vorformlinge sterilisiert werden muss. Damit kann jedoch nicht nur die Menge an Sterilisationsmittel reduziert werden, sondern auch der Prozess deutlich vereinfacht werden. So kann beispielsweise bei der Verwendung von verdampfbaren Sterilisationsmittellösungen (wie z. B. Alkoholen, Chlorwasser, Hypochlorige Säure, Ozon-Lösung, Wasserstoffperoxid, Peroxyessigsäure und anderen) überschüssiges Sterilisationsmittel leicht wieder entfernt werden. Spätestens bei der Erhitzung der Vorformlinge für den Blasprozess entweichen auch die letzten Reste des Sterilisationsmittels, wobei selbstverständlich bei Peroxyden die entsprechenden Sicherheitsaspekte berücksichtigt werden müssen. Eine spätere aufwändige Reinigung und Trocknung der fertigen Gebinde vor dem Abfüllvorgang ist nicht notwendig. Somit ist es trotz des zusätzlichen Aufwandes zur Gewährleistung der Sterilität während des gesamten Produktionsprozesses möglich, die Effizienz deutlich zu steigern.

Wichtig für eine hohe Effizienz ist, dass nur ein möglichst geringer Teil der Vorrichtungen zum Behandeln von Behältnissen die aseptischen Bedingungen ausweist. Da das Aufrechterhalten dieser sterilen Bedingungen recht kostenintensiv ist, hat es sich als vorteilhaft erwiesen, lediglich den Bereich der Vorrichtungen zum Behandeln von Behältnissen steril zu halten, in denen sich die sterilen Vorformlinge oder Gebinde befinden. Eine derartige Vorrichtung ist beispielsweise in WO 2010/020529 A2 offenbart.

In DE 10 2007 017 938 A1 ist eine Blasmaschine offenbart, die sich vollständig in einem sterilen Reinraum (Isolatorraum) befindet. Um die Sterilität auch langfristig im laufenden Betrieb aufrecht halten zu können, sind auf dem Blasrad und entlang des Transportweges der Vorformlinge bzw. Gebinde Strahlungsquellen angeordnet, die die zu sterilisierenden Teile mit Strahlung beaufschlagen, um Keime abzutöten.

Die Verwendung von Gasen oder Aerosolen zur Sterilisation von Reinräumen bietet jedoch entscheidende Vorteile gegenüber einer Sterilisation mittels Strahlung. So ist es insbesondere bei der Verwendung von Sterilisationsgasen durch deren großes Diffusionsvermögen möglich, auch in Bereiche vorzudringen, die für Strahlungsquellen unzugänglich sind. Beispielsweise sind bei der Verwendung von UV-Lampen zur Sterilisation bestimmte Bereiche der Blasstation länger dem von diesen Lampen abgestrahlten Licht ausgesetzt als andere Bereiche. Dies beruht unter anderem auf der Form der einzelnen Blasstationen, die zumindest zeitweise einen Schatten in bestimmte Bereiche werfen. In diesen Bereichen ist es unter Umständen möglich, dass sich Keime ablagern, vermehren und zu Kontaminationen führen.

Aufgabe der Erfindung ist es daher eine Vorrichtung zum Behandeln von Kunststoffbehältnissen, wobei die Vorrichtung mindestens einen beweglichen Träger aufweist, welcher in einen Isolatorraum angeordnet ist, zu dem ein Sterilisationsmittel zuführbar ist, bereit zu stellen, wobei der Isolatorraum aus beweglichen und unbeweglichen Elementen besteht und die Vorrichtung Einrichtungen zum Zuführen eines Sterilisationsmittels in den Isolatorraum aufweist, die jeweils zu diesen Einrichtungen relativbewegliche Teile innerhalb des Isolatorraums durch die Beaufschlagung mit einem Sterilisationsmittel sterilisieren.

Diese Aufgabe wird erfindungsgemäß durch eine Vorrichtung nach Anspruch 1 gelöst.

Eine erfindungsgemäße Vorrichtung zum Behandeln von Kunststoffbehältnissen weist mindestens einen beweglichen Träger auf, welcher in einen Isolatorraum angeordnet ist, zu dem ein Sterilisationsmittel zuführbar ist, wobei der Isolatorraum mindestens zweiteilig ausgebildet ist und eine erste Begrenzung des Isolatorraums während einer Bewegung des beweglichen Trägers positionsfest ist, wohingegen eine zweite Begrenzung des Isolatorraums mit der Vorrichtung bzw. der Blasstation derart verbunden ist, so dass sie dazu geeignet ist, während einer Bewegung des beweglichen Trägers dieser Bewegung zu folgen, wobei die Vorrichtung mindestens eine Einrichtung zum Zuführen des Sterilisationsmittels in den Isolatorraum aufweist, welche dazu geeignet ist, die zu dieser Einrichtung relativbeweglichen innerhalb des Isolatorraums befindlichen Teile der Vorrichtung mit dem Sterilisationsmittel zu beaufschlagen.

Dadurch ist es möglich, das Sterilisationsmittel als Gas oder feinen Nebel (Aerosol) auf die zu desinfizierenden Bereiche der Vorrichtung bzw. der Blasmaschine zu leiten. Es besteht die Möglichkeit, das Sterilisationsmittel auch erwärmt in das Innere des Isolatorraums einzuleiten. Die Einrichtung zum Zuführen des Sterilisationsmittels in den Isolatorraum ist dabei vorzugsweise so ausgebildet, dass sie während der Bewegung der Vorrichtung bzw. des beweglichen Trägers diejenigen Teile innerhalb des Isolatorraums (Reinraums) in etwa gleichmäßig mit Sterilisationsmittel beaufschlagt, die relativbeweglich zu der Einrichtung angeordnet sind. So werden beispielsweise bei einem Blasrad während eines Umlaufs alle in dem Isolatorraum befindlichen Teile des Blasrades an einer stationären Einrichtung zum Zuführen des Sterilisationsmittels in den Isolatorraum vorbeigeführt und mit dem Sterilisationsmittel beaufschlagt. Ebenso ist es möglich, die Einrichtung zum Zuführen des Sterilisationsmittels in dem Isolatorraum beweglich auf dem beweglichen Träger, beispielsweise dem Blasrad anzuordnen, um die feststehenden Teile des Isolatorraums während der Bewegung, beispielsweise eines Umlaufes des Blasrades, mit dem Sterilisationsmittel zu beaufschlagen. Erfindungsgemäß ist eine Vielzahl von Einrichtungen zum Zuführen des Sterilisationsmittels in den Isolatorraum vorgesehen, wobei diese unabhängig voneinander sowohl stationär als auch beweglich gegenüber der mindestens einen Einrichtung zum Aufnehmen der Kunststoffbehältnisse (z. B. einer Blasstation) angeordnet sein können.

Zur Entfernung von Resten des Sterilisationsmittels nach einem Sterilisationsvorgang im Inneren des Isolatorraums, die evtl. in die produzierten, transportierten oder behandelten Kunststoffbehältnisse gelangen könnten, ist es möglich auch andere Substanzen wie beispielsweise eine Waschlösung über die Einrichtung zum Zuführen des Sterilisationsmittels in den Isolatorraum einzubringen. Ebenso ist beispielsweise die sequenzielle Verwendung von mehreren verschiedenen Sterilisationsmitteln (z. B. bakterizide, sporozide, fungizide und viruzide Sterilisationsmittel oder Kombinationen davon) möglich.

Bei der erfindungsgemäßen Vorrichtung handelt es sich insbesondere um eine Blasmaschine. Bei der erfindungsgemäßen Vorrichtung könnte es sich jedoch auch um eine Heizeinrichtung handeln, welche Kunststoffvorformlinge erwärmt oder aber um eine Befüllungseinrichtung zum Befüllen von Behältnissen. Daneben wäre die Erfindung auch für Transporteinrichtungen anwendbar, welche Kunststoffbehältnisse wie Kunststoffvorformlinge oder Kunststoffflaschen transportieren. Allgemein ist die Erfindung bei solchen Anlagen anwendbar, welche bewegliche und insbesondere drehende Teilen sowie einen Reinraum aufweisen.

Erfindungsgemäß weist mindestens eine Einrichtung zum Zuführen des Sterilisationsmittels in den Isolatorraum eine Vielzahl von Düsen auf. Durch diese Düsen ist es einerseits möglich, das Sterilisationsmedium in einer Vorzugsrichtung in den Isolatorraum einzubringen und somit zu gewährleisten, dass auch verhältnismäßig schlecht zugängliche Bereiche des Isolatorraums mit dem Sterilisationsmedium beaufschlagt werden. Außerdem ist es durch diese Düsen möglich, flüssige Sterilisationsmittel und deren Lösungen fein im Inneren des Isolatorraums zu verteilen und ein Aerosol oder Nebel auszubilden. Dieses bleibt längere Zeit stabil, wodurch während einer Bewegung des beweglichen Trägers durch die bei der Bewegung entstehenden Luftverwirbelungen im Inneren des Reinraumes eine äußerst gleichmäßige Verteilung dieser feinen Aerosoltropfen erreicht wird. Damit wird eine gleichmäßige Beaufschlagung der zu sterilisierenden Teile mit dem Sterilisationsmittel gewährleistet. Es ist auch möglich, die Düsen schwenkbar oder drehbar auszuführen, um dem austretenden Sterilisationsmittel eine veränderte bevorzugte Strömungsrichtung zu geben.

In einer weiteren bevorzugten Ausführungsform der Vorrichtung sind der bewegliche Teil der Begrenzung des Isolatorraums (Isolatorraumbegrenzung) und der stationäre Teil der Begrenzung des Isolatorraums mittels einer zwischen diesen Begrenzungen angeordneten Dichtung gegenüber der Umgebung derart abgeschlossen, so dass eine Bewegung dieser Begrenzungen gegenüber einander möglich ist, ein Eindringen von Kontaminationen jedoch ausgeschlossen ist. Prinzipiell kann jeder geeignete Dichtungstyp zur Abgrenzung des Isolatorrauminneren gegenüber der unsterilen Umgebung bei gleichzeitiger beweglicher Lagerung des beweglichen Teils der Isolatorraumbegrenzung gegenüber dem stationären Teil der Isolatorraumbegrenzung verwendet werden. Aufgrund der großen Dichtungsfläche und der ständigen Bewegung, die potentiell Abrieb und damit erhöhten Verschleiß der Dichtungselemente verursacht, sind Dichtungen bevorzugt, die ein Sperrmedium verwenden. Vorzugsweise finden sogenannte Wasserschlösser Anwendung.

Zum möglichst effizienten Sterilisieren der in dem Innereren des Reinraums befindlichen Teile der Vorrichtung haben sich positionsfeste Einrichtungen zum Zuführen des Sterilisationsmittels zu dem Isolatorraum als besonders geeignet erwiesen. Daher sind erfindungsgemäß mindestens einige der Einrichtungen zum Zuführen des Sterilisationsmittels zu dem Isolatorraum positionsfest angeordnet. Diese sind bevorzugt derart angeordnet, dass während eines vollständigen Bewegungszykluses alle im Inneren des Reinraums befindlichen Teile der Vorrichtung die Einrichtungen zum Zuführen des Sterilisationsmittels zu dem Isolatorraum in geringem Abstand passieren und so diese Teile direkt mit dem Sterilisationsmittel beaufschlagt werden können. Die Einrichtungen zum Zuführen des Sterilisationsmittels können dabei an einem stationären Träger angeordnet sein oder etwa auch an einer stationären Begrenzungswand des Isolatorraums.

Nur mit einer einzelnen stationären Einrichtung zum Zuführen des Sterilisationsmittels zu dem Isolatorraum ergibt sich jedoch die Problematik, dass die stationären Teile des Isolatorraums oft nur unzureichend mit dem Sterilisationsmittel beaufschlagt werden. In besonders aufwändig ausgebildeten Ausführungsformen von Vorrichtungen zum Behandeln von Kunststoffbehältnissen ist auch durch das bereits beschriebene feine Verteilen des Sterilisationsmittels als Nebel im Inneren des Isolatorraums und eine anschließende Verwirbelung dieses Nebels nicht unbedingt eine ausreichend gleichmäßige Verteilung gewährleistet. Eine ausreichende Sterilisierung der unzugänglichen Bereiche könnte beispielsweise durch gelegentliche separate Sterilisierung erreicht werden. Da dies sehr zeitaufwändig ist und einen Stillstand der gesamten Anlage erfordert, sind Einrichtungen vorteilhaft, die auch eine ausreichende Sterilisierung der stationären Teile gewährleisten.

In einer bevorzugten Ausführungsform der Vorrichtung sind daher mindestens einige der Einrichtungen zum Zuführen des Sterilisationsmittels zu dem Isolatorraum beweglich angeordnet. Befinden sich diese beweglich angeordneten Einrichtungen zum Zuführen des Sterilisationsmittels zu dem Isolatorraum beispielsweise an oder auf dem beweglichen Träger (z. B. dem Blasrad), passieren sie während eines Bewegungszykluses den gesamten stationären Teil der Isolatorraumbegrenzung und können diesen mit Sterilisationsmitteln beaufschlagen. Bevorzugt sind auch diese Einrichtungen zum Zuführen des Sterilisationsmittels zu dem Isolatorraum als Düsen ausgeführt. Vorteilhaft sind die besagten Einrichtungen zum Zuführen des Sterilisationsmittels derart angeordnet, dass sie das Sterilisationsmittel wenigstens entlang der vollständigen Höhe des Isolatorraums aufbringen.

Bei einer derartigen Ausführung ergibt sich jedoch die Problematik der kontinuierlichen Zuführung des Sterilisationsmittels zu den beweglichen Einrichtungen zum Zuführen des Sterilisationsmittels zu dem Isolatorraum. Erfindungsgemäß sind daher die beweglich angeordneten Einrichtungen zum Zuführen des Sterilisationsmittels in den Isolatorraum mit einem Drehverteiler in Verbindung, welcher dazu geeignet ist, das Sterilisationsmittel zu den Einrichtungen zuzuführen. Somit ist auf einfache Weise eine kontinuierliche ausreichende Versorgung der Einrichtungen zum Zuführen des Sterilisationsmittels in den Isolatorraum mit Sterilisationsmedium gewährleistet.

In einer weiteren bevorzugten Ausführungsform der Vorrichtung weist die Vorrichtung ein zentrales Reservoir für das Sterilisationsmittel auf. Aus diesem Reservoir können sowohl die beweglichen als auch die stationären Einrichtungen zum Zuführen des Sterilisationsmittels in den Isolatorraum mit dem Sterilisationsmittel versorgt werden. Die Zuführung zu den beweglichen Einrichtungen erfolgt wie bereits erwähnt bevorzugt mittels eines Drehverteilers.

Bevorzugt handelt es sich bei dem beweglichen Träger der Vorrichtung um eine annähernd kreisrunde Vorrichtung. In einer bevorzugten Ausführungsform der Vorrichtung ist daher der bewegliche Träger ein Blasrad mit mindestens einer Blasstation, welches zumindest teilweise im Inneren des Isolatorraums angeordnet ist. Diese Ausführungsform als annähernd kreisrundes Blasrad vereinfacht die Geometrie des Isolatorraums und reduziert gleichzeitig Bereiche im Inneren des Isolatorraums, die für das Sterilisationsmittel schwer zugänglich sind. Vorteilhaft weist der Isolatorraum eine ring- oder torusförmige Gestalt auf, wobei die Blasstationen zumindest teilweise innerhalb des Isolatorraums transportiert werden.

Wie bereits beschrieben handelt es sich bei den Sterilisationsmitteln bevorzugt um leicht verteilbare Substanzen. In einer bevorzugten Ausführungsform liegt das Sterilisationsmittel zum Zeitpunkt des Einspritzens in den Isolatorraum gasförmig oder flüssig vor. Sowohl gasförmig als auch flüssig (bevorzugt als feinverteiltes Aerosol) ist das Sterilisationsmittel leicht verteilbar. Insbesondere während einer Bewegung des Trägers, an dem die einzelnen Einrichtungen zum Behandeln von Kunststoffbehältnissen angeordnet sind, kann es daher durch die entstehenden Luftverwirbelungen fein verteilt werden. Dadurch ist eine ausreichende Beaufschlagung mit dem Sterilisationsmittel auch für die Bereiche im Inneren des Isolatorraums gewährleistet, die bei einer Einspritzung des Sterilisationsmittels nur schwer direkt zugänglich sind.

Zur weiteren Verteilung des Sterilisationsmittels im Inneren des Isolatorraums der Vorrichtung ist es in bestimmten Fällen notwendig, für zusätzliche Luftbewegungen zu sorgen. Daher weist in einer weiteren bevorzugten Ausführungsform der Isolatorraum Eingänge für Druckluft auf, die dazu geeignet sind, eine Hochdruckreinigung des Isolatorraums zu ermöglichen. Ebenso ist es durch diese Eingänge möglich, im Inneren des Isolatorraums einen leichten Überdruck zu erzeugen, der verhindert, dass unsteriles Gas in das Innere des Isolatorraums diffundiert und dieses so kontaminiert.

Ein weiterer wesentlicher Aspekt der Erfindung ist ein Verfahren gemäß Anspruch 10 zur Sterilisierung eines Isolatorraumes einer Vorrichtung zum Behandeln von Kunststoffbehältnissen mit mindestens einen beweglichen Träger, welcher in einen Isolatorraum angeordnet ist, zu dem ein Sterilisationsmittel zugeführt wird und wobei der Isolatorraum mindestens zweiteilig ausgebildet ist und eine erste Begrenzung (Begrenzungseinrichtung) des Isolatorraums sich während einer Bewegung des beweglichen Trägers nicht bewegt, wohingegen eine zweite Begrenzung (Begrenzungseinrichtung) des Isolatorraums mit der Vorrichtung derart verbunden ist, dass sie während einer Bewegung des beweglichen Trägers dieser Bewegung folgt.

Durch dieses Verfahren ist es auf sehr einfache und effiziente Weise möglich, das Innere eines Isolatorraums mit Sterilisationsmittel zu beaufschlagen. Während eines Bewegungszykluses des beweglichen Trägers können so nahezu alle gegenüber der Zuführeinrichtung relativbeweglichen Teile mit dem Sterilisationsmittel beaufschlagt werden. Vorteilhaft wird die besagte Relativbewegung durch eine Bewegung eines Trägers bewirkt, an dem die Vorrichtung zum Behandeln von Kunststoffbehältnissen (z. B. eine oder mehrere Blasstationen) angeordnet ist. Ebenso besteht die Möglichkeit, das Sterilisationsmittel auch erhitzt in das Innere des Isolatorraums einzuleiten. Beispielsweise können H₂O₂, Peroxyessigsäure oder andere Sterilisationsmittel oder deren wässrige Lösungen vor der Einleitung in den Isolatorraum in die Gasphase überführt werden.

Weitere Vorteile, Ziele und Eigenschaften vorliegender Erfindung werden anhand nachfolgender Beschreibung anliegender Zeichnung erläutert, in welcher beispielhaft eine erfindungsgemäße Vorrichtung am Beispiel einer Blasmaschine mit wenigstens einer Blasstation zum Umformen von Kunststoffvorformlingen zu Kunststoffbehältnissen dargestellt ist, wobei die Blasstation an einem beweglichen Träger angeordnet ist und die Blasmaschine einen Isolatorraum aufweist, zu dem ein Sterilisationsmittel zuführbar ist.

Es zeigen:
- Fig. 1: eine Blasstation nach dem Stand der Technik,
- Fig. 2: eine schematische Aufsicht auf eine Blasmaschine mit einer Zuführeinrichtung, einer Abführeinrichtung und einem Isolatorraum, in den mittels geeigneter Einrichtungen ein Sterilisationsmittel zuführbar ist;
- Fig. 3: eine schematische Darstellung einer Blasstation, die sich teilweise im Inneren eines Isolatorraums befindet, wobei dem Isolatorraum mittels einer beweglichen Einrichtung Sterilisationsmittel zuführbar ist; und
- Fig. 4:: eine schematische Darstellung einer Blasstation, die sich teilweise im Inneren eines Isolatorraums befindet, wobei dem Isolatorraum mittels einer stationären Einrichtung Sterilisationsmittel zuführbar ist.

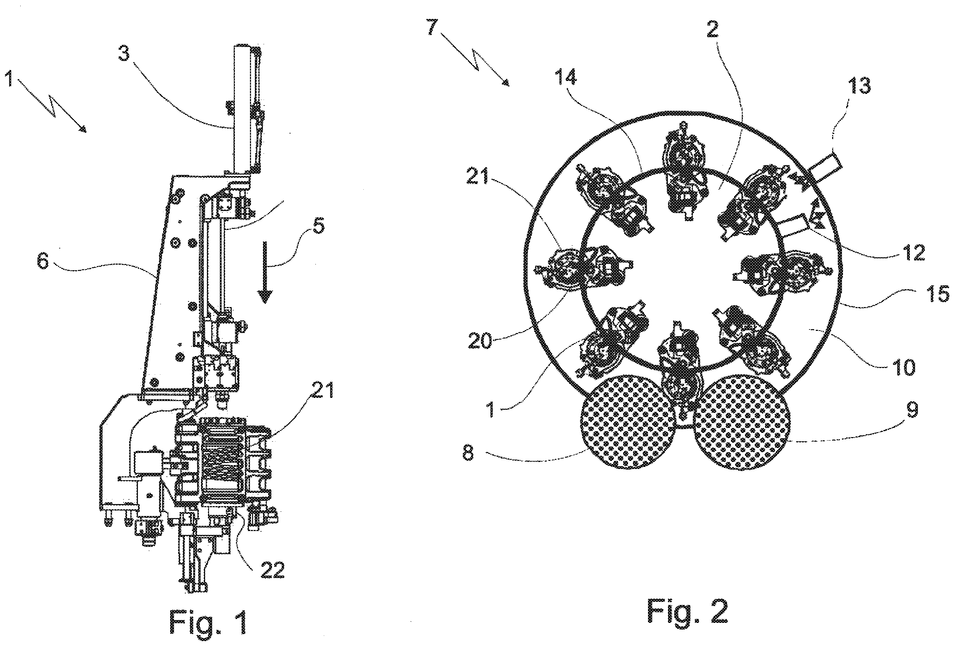

Fig. 1 zeigt eine Blasstation 1 nach dem Stand der Technik zum Umformen von Kunststoffvorformlingen zu Kunststoffbehältnissen. Eine Vielzahl derartiger Blasstationen 1 ist dabei auf einem (nicht gezeigten) drehbaren Blasrad angeordnet. Das Bezugszeichen 3 in Fig. 1 zeigt eine Antriebseinrichtung 3, welche zum Betätigen der Reckstange 4, d. h. zum Bewegen der Reckstange 4 in der Dehnungsrichtung 5 dient. Das Bezugszeichen 6 bezieht sich auf einen Träger 6, an dem die Reckstangenanordnung 4 angeordnet ist. Die Blasstation weist dabei zwei Seitenteile 20, 21 (vgl. Fig. 2) sowie ein Bodenteil 22 auf, welche gemeinsam einen Hohlraum ausbilden, in dem Kunststoffvorformlinge in Kunststoffbehältnisse expandierbar sind.

Fig. 2 zeigt eine schematische Aufsicht auf eine Blasmaschine 7 mit einer Zuführeinrichtung 9, einer Abführeinrichtung 8, einer Vielzahl von Blasstationen 1 und einem Isolatorraum 10, in den mittels geeigneter Einrichtungen 12, 13 ein Sterilisationsmittel zuführbar ist. Der bewegliche Träger 2 der Blasmaschine 7 auf dem die Blasstationen 1 angeordnet sind, ist als kreisrundes Blasrad 2 ausgebildet. An beispielhaften Positionen sind in Figur 2 jeweils eine bewegliche 12 und eine stationäre 13 Einrichtung zum Zuführen des Sterilisationsmittels gezeigt. Während einer Rotation des radial innenliegenden Teils 14 des Isolatorraums 10 passieren alle beweglichen Elemente (wie beispielsweise die Blasstationen 1) die stationäre Einrichtung 13 zum Zuführen des Sterilisationsmittels und können mit dem Sterilisationsmittel beaufschlagt werden. Die bewegliche Einrichtung 12 zum Zuführen des Sterilisationsmittels kann während eines Umlaufes den stationären, radial außenliegenden Teil 15 des Isolatorraums 9 mit dem Sterilisationsmittel beaufschlagen. Die besagte Einrichtung 12 zum Zuführen des Sterilisationsmittels ist dabei zwischen zwei Blasstationen 1 angeordnet.

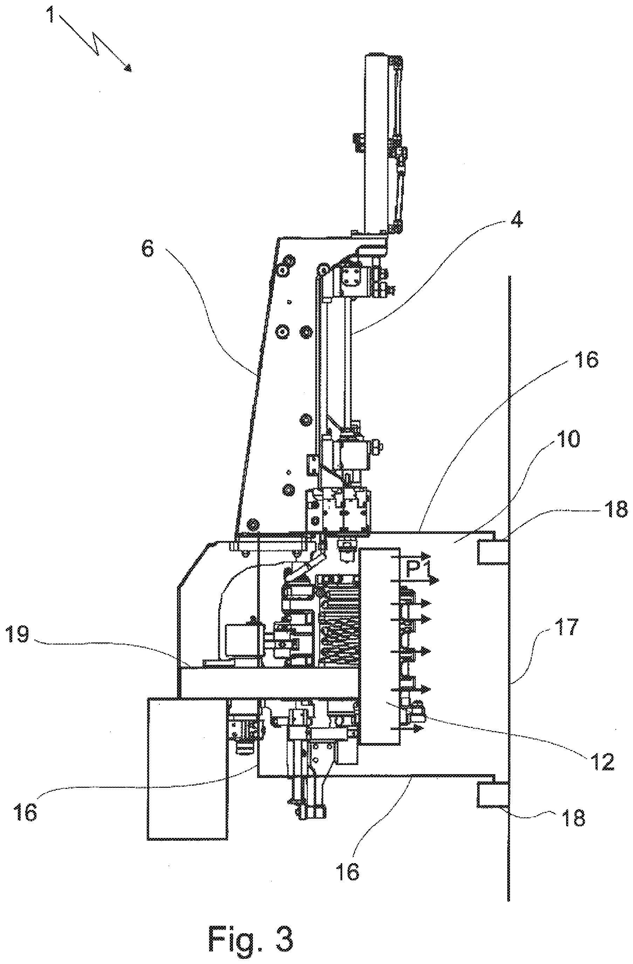

Fig. 3 zeigt eine schematische Darstellung einer Blasstation 1, die sich teilweise im Inneren eines Isolatorraums 9 befindet, wobei dem Isolatorraum 9 mittels einer beweglichen Einrichtung 12 Sterilisationsmittel zuführbar sind (Pfeile P1). In dem gezeigten Beispiel ist der Isolatorraum 9 auf drei Seiten durch Begrenzungen 16 abgeschlossen, die Bewegungen des Blasrades 2 folgen. Gegenüber der stationären Begrenzung 17 des Isolatorraums 9 sind sie mittels Dichtungen 18 beweglich, aber gasdicht gelagert. Ebenso ist die Reckstange 4 mittels einer Dichtung (beispielsweise einem Faltenbalg) gegenüber dem Isolatorraum 9 abgeschirmt, um bei einem Absenken der Reckstange 4 aus dem unsterilen Bereich in das Innere des Kunststoffvorformlings und damit in den sterilen Bereich (nämlich das Innere des Isolatorraums 9), die Sterilität des Isolatorraums 9 zu erhalten. Bevorzugt in den Zwischenräumen zwischen Blasstationen 1 befindet sich die mindestens eine Einrichtung 12, 13 zum Zuführen des Sterilisationsmittels.

Über ein Rohrleitungssystem 19 kann dieser Einrichtung 12, 13 das Sterilisationsmittel zugeführt werden. Optional befinden sich Dosier- oder Drosseleinrichtungen im Leitungssystem.

Über Austrittsöffnungen kann das Sterilisationsmittel aus der Einrichtung 12, 13 austreten und ins Innere des Isolatorraums 9 bzw. auch an die Innenoberflächen der Begrenzungen 16, 17 gelangen. Über die Form und Ausrichtung der Austrittsöffnungen kann die Richtung vorgegeben werden, in der das Sterilisationsmittel abgegeben wird.

Ebenso kann über (nicht gezeigte) Düsen auch der Grad der Zerstäubung vorgegeben werden, so dass es möglich ist, ein feines Aerosol des Sterilisationsmittels zu erzeugen, welches längere Zeit in der Gasphase verbleibt, ohne sich vollständig abzusetzen. Um nach einem Sterilisationsvorgang zu vermeiden, dass Reste des Aerosols verbleiben und evtl. in das Innere der produzierten Kunststoffbehältnisse gelangen, ist es möglich, auch andere Substanzen wie beispielsweise eine Waschlösung über die Einrichtung 12, 13 zum Zuführen des Sterilisationsmittels in den Isolatorraum einzubringen. Auch die Verwendung von mehreren verschiedenen Sterilisationsmitteln (z. B. bakterizide, sporozide, fungizide und viruzide Sterilisationsmittel oder Kombinationen davon) ist somit möglich.

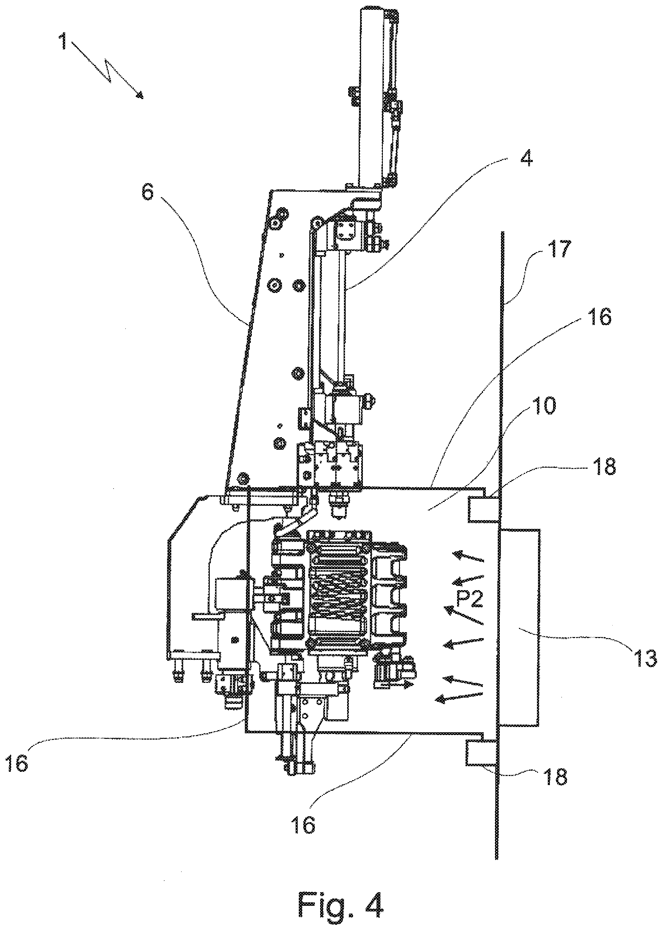

Fig. 4 zeigt eine schematische Darstellung einer Blasstation 1, die sich teilweise im Inneren eines Isolatorraums 9 befindet, wobei dem Isolatorraum 9 mittels einer stationären Einrichtung 13 Sterilisationsmittel zuführbar sind (Pfeile P2). Wie auch in Figur 3 gezeigt ist der Isolatorraum 9 auf drei Seiten durch Begrenzungen 16 abgeschlossen, die den Bewegungen des Blasrades 2 folgen. Im Bereich der stationären Begrenzung 17 des Isolatorraums 9 ist eine stationäre Einrichtung 13 zum Zuführen des Sterilisationsmittels angeordnet. Dieser kann über ein (nicht gezeigtes) Rohrleitungssystem das Sterilisationsmittel zugeführt werden. Gegenüber den beweglichen Begrenzungen 16 des Isolatorraums 9 ist die stationäre Begrenzung 17 mittels mindestens einer Dichtung 18 derart gasdicht gelagert, dass eine Bewegung des Blasrades 2 möglichst ohne größere Energieverluste möglich ist. Über Austrittsöffnungen kann das Sterilisationsmittel aus der stationären Einrichtung 13 austreten und direkt auf die während einer Rotation des Blasrades 2 an dieser vorbeigeführten beweglichen Teile im Inneren des Isolatorraums 9 aufgebracht werden. Auch bei den stationären Austrittsöffnungen kann es sich um Düsen handeln, die es ermöglichen, die Richtung vorzugegeben, in der das Sterilisationsmittel versprüht wird. Auch der Grad der Zerstäubung kann über die Art oder die Einstellung der Düsen vorgegeben werden. Es ist auch möglich, die Düsen schwenkbar oder drehbar auszuführen, um dem austretenden Sterilisationsmittel eine veränderte bevorzugte Strömungsrichtung zu geben.

Die Anmelderin behält sich vor, sämtliche in den Anmeldungsunterlagen offenbarten Merkmale als erfindungswesentlich zu beanspruchen, sofern diese einzeln oder in Kombination gegenüber dem Stand der Technik neu sind.

### Bezugszeichenliste

- 1: Blasstation
- 2: Träger / Blasrad
- 3: Antriebseinrichtung
- 4: Reckstange
- 5: Dehnungsrichtung
- 6: Träger
- 7: Vorrichtung zum Behandeln von Kunststoffbehältnissen (z. B. Blasmaschine)
- 8: Abführeinrichtung
- 9: Zuführeinrichtung
- 10: Isolatorraum
- 12: bewegliche Einrichtung zum Zuführen des Sterilisationsmittels
- 13: stationäre Einrichtung zum Zuführen des Sterilisationsmittels
- 14: radial innenliegender Teils des Isolatorraums
- 15: radial außenliegender Teil des Isolatorraums
- 16: bewegliche Begrenzungen
- 17: stationäre Begrenzung
- 18: Dichtung
- 19: Rohrleitungssystem
- 20: erstes Seitenteil
- 21: zweites Seitenteil
- 22: Bodenteil

## Patentansprüche

1. Vorrichtung (7) zum Behandeln von Kunststoffbehältnissen, wobei die Vorrichtung (7) mindestens einen beweglichen Träger (2) aufweist, welcher in einen Isolatorraum (10) angeordnet ist, zu dem ein Sterilisationsmittel zuführbar ist, wobei der Isolatorraum (10) mindestens zweiteilig ausgebildet ist und eine erste Begrenzung (15) des Isolatorraums
(9) während einer Bewegung des beweglichen Trägers (2) positionsfest ist, wohingegen eine zweite Begrenzung (14) des Isolatorraums (10) mit der Vorrichtung (7) derart verbunden ist, so dass sie dazu geeignet ist, während einer Bewegung des beweglichen Trägers (2) dieser Bewegung zu folgen, wobei die Vorrichtung (7) mindestens eine Einrichtung (12, 13) zum Zuführen des Sterilisationsmittels in den Isolatorraum (10) aufweist, welche dazu geeignet ist, die zu dieser Einrichtung (12, 13) relativbeweglichen innerhalb des Isolatorraums (10) befindlichen Teile der Vorrichtung (7) mit dem Sterilisationsmittel zu beaufschlagen,
wobei die mindestens eine Einrichtung (12) zum Zuführen des Sterilisationsmittels zu dem Isolatorraum (10) mit einem Drehverteiler verbunden ist,
und wobei
die Einrichtung (12,13) zum Zuführen des Sterilisationsmediums in den Isolatorraum (10) eine Vielzahl von Düsen aufweist,
**dadurch gekennzeichnet, dass**
mindestens eine der Einrichtungen zum Zuführen des Sterilisationsmittels zu dem Isolatorraum (10) positionsfest angeordnet ist und mindestens eine der Einrichtungen (12) zum Zuführen des Sterilisationsmittels zu dem Isolatorraum (10) beweglich angeordnet ist.

2. Vorrichtung (7) nach Anspruch 1,
**dadurch gekennzeichnet, dass**
die zu dem Isolatorraum (10) beweglich angeordnete Einrichtung (12) zum Zuführen des Sterilisationsmittels in den Isolatorraum (10) eine Vielzahl von Düsen aufweist.

3. Vorrichtung (7) nach einem der vorangegangenen Ansprüche,
**dadurch gekennzeichnet, dass**
der bewegliche Teil der Begrenzung (14) des Isolatorraums (10) und der stationäre Teil der Begrenzung (15) des Isolatorraums (10) mittels einer zwischen diesen Begrenzungen angeordneten Dichtung (18) gegenüber der Umgebung derart abgeschlossen sind, dass eine Bewegung dieser Begrenzungen (14, 15) gegenüber einander möglich ist, ein Eindringen von Kontaminationen jedoch ausgeschlossen ist.

4. Vorrichtung (7) nach einem der vorangegangenen Ansprüche,
**dadurch gekennzeichnet, dass**
mindestens einige der Einrichtungen (12) zum Zuführen des Sterilisationsmittels zu dem Isolatorraum (10) beweglich angeordnet sind.

5. Vorrichtung (7) nach einem der vorangegangenen Ansprüche,
**dadurch gekennzeichnet, dass**
die beweglich angeordneten Einrichtungen (12) zum Zuführen des Sterilisationsmittels in den Isolatorraum (10) mit einem Drehverteiler in Verbindung sind, welcher dazu geeignet ist, das Sterilisationsmittel zu den Einrichtungen (12) zuzuführen.

6. Vorrichtung (7) nach einem der vorangegangenen Ansprüche,
**dadurch gekennzeichnet, dass**.
die Vorrichtung (7) ein zentrales Reservoir für das Sterilisationsmittel aufweist.

7. Vorrichtung (7) nach einem der vorangegangenen Ansprüche,
**dadurch gekennzeichnet, dass**.
der bewegliche Träger (2) ein Blasrad (2) mit mindestens einer Blasstation (1) ist, welches zumindest teilweise im Inneren des Isolatorraums (10) angeordnet ist.

8. Vorrichtung (7) nach einem der vorangegangenen Ansprüche,
**dadurch gekennzeichnet, dass**
die Einrichtungen (12, 13) zum Zuführen des Sterilisationsmittels in den Isolatorraum (10) zur gasförmigen oder flüssigen Zuführung des Sterilisationsmittels in den Isolatorraum geeignet sind.

9. Vorrichtung (7) nach einem der vorangegangenen Ansprüche, wobei der Isolatorraum (10) Eingänge für Druckluft aufweist, die dazu geeignet sind, eine Hochdruckreinigung des Isolatorraums (10) zu ermöglichen.

10. Verfahren zur Sterilisierung eines Isolatorraumes (10) einer Vorrichtung (7) zum Behandeln von Kunststoffbehältnissen mit mindestens einem beweglichen Träger (2) welcher in einem Isolatorraum (10) angeordnet ist, zu dem ein Sterilisationsmittel zugeführt wird, wobei der Isolatorraum (10) mindestens zweiteilig ausgebildet ist und eine erste Begrenzung (15) des Isolatorraums (10) sich während einer Bewegung der Vorrichtung (7) nicht bewegt, wohingegen eine zweite Begrenzung (14) des Isolatorraums (10) mit der Vorrichtung (7) derart verbunden ist, so dass sie während einer Bewegung der Vorrichtung (7) dieser Bewegung folgt, wobei mindestens eine Einrichtung (12, 13) zum Zuführen des Sterilisationsmittels in den Isolatorraum (10) vorgesehen ist, mit welcher die zu dieser Einrichtung (12, 13) relativbeweglichen innerhalb des Isolatorraums (10) befindlichen Teile der Blasmaschine (7) mit dem Sterilisationsmittel beaufschlagt werden, wobei das Sterilisationsmittel mit mindestens einer Einrichtung (12) zum Zuführen des Sterilisationsmittels zu dem Isolatorraum (10) zugeführt wird, die mit einem Drehverteiler verbunden ist
wobei
die Einrichtung (12,13) zum Zuführen des Sterilisationsmediums in den Isolatorraum (10) eine Vielzahl von Düsen aufweist,
**dadurch gekennzeichnet, dass**
mindestens eine der Einrichtungen zum Zuführen des Sterilisationsmittels zu dem Isolatorraum (10) positionsfest angeordnet ist und mindestens eine der Einrichtungen (12) zum Zuführen des Sterilisationsmittels zu dem Isolatorraum (10) beweglich angeordnet ist.

11. Verfahren zur Sterilisierung eines Isolatorraumes (10) einer Vorrichtung (7) zum Behandeln von Kunststoffbehältnissen nach Anspruch 10,
**dadurch gekennzeichnet, dass**
das Sterilisationsmittel zum Zeitpunkt des Einspritzens in den Isolatorraum (10) diesem gasförmig oder flüssig zugeführt wird.

## Claims

1. An apparatus (7) for the treatment of plastics material containers, wherein the apparatus (7) has at least one movable support (2) which is arranged in an isolator room (10) to which a sterilization agent is capable of being supplied, wherein the isolator room (10) is constructed in at least two parts and a first boundary (15) of the isolator room (10) is fixed in position during a movement of the movable support (2), whereas a second boundary (14) of the isolator room (10) is connected to the apparatus (7) in such a way that it is suitable, during a movement of the movable support (2), for following this movement, wherein the apparatus (7) has at least one device (12, 13) for supplying the sterilization agent into the isolator room (10), which device (12, 13) is suitable for acting with the sterilization agent upon the parts of the apparatus (7) movable relative to this device (12, 13) and situated inside the isolator room (10),
wherein the at least one device (12) for supplying the sterilization agent to the isolator room (10) is connected to a rotary distributor,
and wherein
the device (12, 13) for supplying the sterilization agent into the isolator room (10) has a plurality of nozzles,
**characterized in that**
at least one of the devices for supplying the sterilization agent to the isolator room (10) is arranged fixed in their position and at least one of the devices (12) for supplying the sterilization agent to the isolator room (10) is arranged in a movable manner.

2. An apparatus (7) according to claim 1,
**characterized in that**
the device (12) being arranged movably to the isolator room (10) for supplying the sterilization agent into the isolator room (10) has a plurality of nozzles.

3. An apparatus (7) according to one of the preceding claims,
**characterized in that**
the movable part of the boundary (14) of the isolator room (10) and the stationary part of the boundary (15) of the isolator room (10) are closed off from the environment by means of a seal (18) arranged between these boundaries, in such a way that a movement of these boundaries (14, 15) with respect to each other is possible, but penetration by contamination is eliminated.

4. An apparatus (7) according to any one of the preceding claims,
**characterized in that**
at least some of the devices (12) for supplying the sterilization agent to the isolator room (10) are arranged in a movable manner.

5. An apparatus (7) according to any one of the preceding claims,
**characterized in that**
the movably arranged devices (12) for supplying the sterilization agent into the isolator room (10) are connected to a rotary distributor which is suitable for supplying the sterilization agent to the devices (12).

6. An apparatus (7) according to any one of the preceding claims,
**characterized in that**
the apparatus (7) has a central reservoir for the sterilization agent.

7. An apparatus (7) according to any one of the preceding claims,
**characterized in that**
the movable support (2) is a blowing wheel (2) with at least one blowing station (1), which is arranged at least in part in the interior of the isolator room (10).

8. An apparatus (7) according to any one of the preceding claims,
**characterized in that**
the devices (12, 13) for supplying the sterilization agent into the isolator room (10) are suitable for gaseous or liquid supply of the sterilization agent into the isolator room.

9. An apparatus (7) according to any one of the preceding claims,
**characterized in that**
the isolator room (10) has inlets for compressed air, which are suitable for permitting a high-pressure cleaning of the isolator room (10).

10. A method of sterilizing an insulator room (10) of an apparatus (7) for the treatment of plastics material containers, with at least one movable support (2) which is arranged in an insulator room (10) to which a sterilization agent is supplied, wherein the isolator room (10) is designed in at least two parts and a first boundary (15) of the isolator room (10) does not move during a movement of the apparatus (7), whereas a second boundary (14) of the isolator room (10) is connected to the apparatus (7) in such a way that during a movement of the apparatus (7) it follows this movement, wherein at least one device (12, 13) is provided for supplying the sterilization agent into the isolator room (10), by which the parts of the blow moulding machine (7) movable relative to this device (12, 13) and present inside the isolator room (10) are acted upon with the sterilization agent, wherein the sterilization agent is supplied to the isolator room (10) with at least one device (12) for supplying the sterilization agent, which device is connected to a rotary distributor,
wherein
the device (12, 13) for supplying the sterilization agent into the isolator room (10) has a plurality of nozzles,
**characterized in that**
at least one of the devices for supplying the sterilization agent to the isolator room (10) is arranged fixed in their position and at least one of the devices (12) for supplying the sterilization agent to the isolator room (10) is arranged in a movable manner.

11. A method of sterilizing an insulator room (10) of an apparatus (7) for the treatment of plastics material containers according to claim 10,
**characterized in that**
the sterilizing agent is supplied to the isolator room (10) in gaseous or liquid form at the time of injection into the isolator room (10).

## Revendications

1. Dispositif (7) pour le traitement de contenants en matière plastique, le dispositif (7) comprenant au moins un support (2) mobile, lequel est agencé dans une chambre isolante (10) à laquelle un agent stérilisant peut être amené, la chambre isolante (10) étant réalisée au moins en deux parties et une première délimitation (15) de la chambre isolante (10) étant fixée en position pendant un déplacement du support (2) mobile, tandis qu'une deuxième délimitation (14) de la chambre isolante (10) est reliée au dispositif (7) de manière à être adaptée à suivre le déplacement du support (2) mobile pendant un déplacement de ce dernier, le dispositif (7) comprenant au moins un système (12, 13) destiné à amener l'agent stérilisant dans la chambre isolante (10), lequel système est adapté à appliquer l'agent stérilisant sur des parties du dispositif (7) situées à l'intérieur de la chambre isolante (10) et mobiles par rapport à ce système (12, 13),
le ou les systèmes (12) destinés à amener l'agent stérilisant à la chambre isolante (10) étant reliés à un distributeur rotatif,
et
le système (12, 13) destiné à amener l'agent stérilisant dans la chambre isolante (10) comprenant une pluralité de buses,
**caractérisé en ce que**
au moins l'un des systèmes destiné à amener l'agent stérilisant à la chambre isolante (10) est monté fixe en position et au moins l'un des systèmes (12) destiné à amener l'agent stérilisant à la chambre isolante (10) est monté mobile.

2. Dispositif (7) selon la revendication 1,
**caractérisé en ce que**
le système (12) monté mobile par rapport à la chambre isolante (10) et destiné à amener l'agent stérilisant dans la chambre isolante (10) comprend une pluralité de buses.

3. Dispositif (7) selon l'une quelconque des revendications précédentes,
**caractérisé en ce que**
la partie mobile de la délimitation (14) de la chambre isolante (10) et la partie fixe de la délimitation (15) de la chambre isolante (10) sont fermées par rapport à l'environnement au moyen d'un joint (18) agencé entre ces délimitations, de telle manière qu'un déplacement de ces délimitations (14, 15) l'une par rapport à l'autre est possible, mais qu'une pénétration de contaminants est exclue.

4. Dispositif (7) selon l'une quelconque des revendications précédentes,
**caractérisé en ce que**
au moins certains des systèmes (12) destinés à amener l'agent stérilisant à la chambre isolante (10) sont montés mobiles.

5. Dispositif (7) selon l'une quelconque des revendications précédentes,
**caractérisé en ce que**
les systèmes (12) montés mobiles destinés à amener l'agent stérilisant dans la chambre isolante (10) sont reliés à un distributeur rotatif, lequel est adapté à amener l'agent stérilisant aux systèmes (12).

6. Dispositif (7) selon l'une quelconque des revendications précédentes,
**caractérisé en ce que**
le dispositif (7) comprend un réservoir central pour l'agent stérilisant.

7. Dispositif (7) selon l'une quelconque des revendications précédentes,
**caractérisé en ce que**
le support (2) mobile est une roue de soufflage (2) comprenant au moins une machine de soufflage (1), laquelle est agencée au moins en partie à l'intérieur de la chambre isolante (10).

8. Dispositif (7) selon l'une quelconque des revendications précédentes,
**caractérisé en ce que**
les systèmes (12, 13) destinés à amener l'agent stérilisant dans la chambre isolante (10) sont adaptés à l'amenée gazeuse ou liquide de l'agent stérilisant dans la chambre isolante.

9. Dispositif (7) selon l'une quelconque des revendications précédentes,
**caractérisé en ce que**
la chambre isolante (10) comprend des entrées pour de l'air comprimé, qui sont adaptées à permettre un nettoyage haute pression de la chambre isolante (10).

10. Procédé de stérilisation d'une chambre isolante (10) d'un dispositif (7) pour le traitement de contenants en matière plastique comprenant au moins un support (2) mobile, lequel est agencé dans une chambre isolante (10) à laquelle un agent stérilisant est amené, la chambre isolante (10) étant réalisée au moins en deux parties et une première délimitation (15) de la chambre isolante (10) étant immobile pendant un déplacement du dispositif (7), tandis qu'une deuxième délimitation (14) de la chambre isolante (10) est reliée au dispositif (7) de manière à suivre le déplacement du dispositif (7) pendant un déplacement de ce dernier, elle suit ledit déplacement, au moins un système (12, 13) destiné à amener l'agent stérilisant dans la chambre isolante (10) étant prévu, système au moyen duquel l'agent stérilisant est appliqué sur des parties de la machine de soufflage (7) situées à l'intérieur de la chambre isolante (10) et mobiles par rapport à ce système (12, 13), l'agent stérilisant étant amené à la chambre isolante (10) au moyen d'au moins un système (12) qui est destiné à amener l'agent stérilisant et qui est relié à un distributeur rotatif,
le système (12, 13) destiné à amener l'agent stérilisant dans la chambre isolante (10) comprenant une pluralité de buses,
**caractérisé en ce que**
au moins l'un des systèmes destinés à amener l'agent stérilisant à la chambre isolante (10) est monté fixe en position et au moins l'un des systèmes (12) destinés à amener l'agent stérilisant à la chambre isolante (10) est monté mobile.

11. Procédé de stérilisation d'une chambre isolante (10) d'un dispositif (7) pour le traitement de contenants en matière plastique selon la revendication 10,
**caractérisé en ce que**
l'agent stérilisant, au moment de l'injection dans la chambre isolante (10), est amené à celle-ci sous forme gazeuse ou liquide.
